# EUROPEAN PATENT APPLICATION

(11) **EP 2 409 719 A1**
(43) Date of publication of application: **25.01.2012**
(21) Application number: 10750392.2
(22) Date of filing: 10.03.2010
(51) Int. Cl.: A61L 15/56, A61F 13/42, A61F 13/47

(54) **INCONTINENCE DETECTOR**

(30) Priority: 13.03.2009 ES 200900705
(71) Applicant: Echeverría Salinas, María Teresa, 46021 Valencia (ES)
(72) Inventor: Echeverría Salinas, María Teresa, 46021 Valencia (ES)
(74) Representative: Cabinet Plasseraud
(86) International application number: PCT/ES2010/000099
(87) International publication number: WO 2010/103142

(57) **Abstract**

Incontinence detector, that comprises an impermeable and breathable sheet (1) on which is spread, on one or both faces, a moisture-reactive product (2), said sheet (1) being wrapped in a covering (3) of structured non-woven microfibre sheet material.

## Description

### Field of the Art

The present invention relates to the incontinence control of human bodily flows, proposing for such purpose a detector which allows warning the affected person when the incontinent flow evacuation happens.

### State of the Art

The incontinence of flows such as urine, menstruation, etc., is a problem affecting many people, with the consequence of uncomfortable insecurity for the affected people due to the lack of self-control and the negative consequences derived therefrom for a normal social living.

As a solution to this, electrical operating apparatuses have been developed, the operational capacity of which is based on the production of an audible signal upon detecting the presence of moisture.

Said apparatuses are uncomfortable for the user due to the size and the cable connections which they have, limiting the freedom of movements of the person, such that their use is virtually restricted to night use and even then with considerable difficulties.

Such apparatuses also do not protect the privacy of the user since the audible warning signal produced is a consequence which is exactly opposite the privacy purpose, which continues generating psychological consequences for the users.

### Object of the Invention

The invention proposes a detector which practically and advantageously solves the mentioned problem since it offers features which eliminate the discomfort of use further limiting the reception of the signals due to the incontinence effect to the user himself/herself.

This detector object of the invention consists of an element formed by a carrier sheet having a product thermally reactive with moisture, such as sodium thiosulfate or any other product with the same reaction.

The carrier sheet having the reactive product is formed by an impermeable and breathable material, such as polyethylene fibre, the reactive product being incorporated on said sheet such that it is spread over the surface of the same.

To this end the reactive product can be included in a compartmentalization by way of bags or meshes made on the carrier sheet, or by means of impregnating said sheet, such that the particles of the product are retained between the fibres of the sheet. The reactive product can thus be on one or both faces on the carrier sheet depending on the detection sensitivity desired.

The carrier sheet having the reactive product can further include an absorbent material and areas impregnated with adhesive being arranged on the same for fixing it during use directly on clothing. Nevertheless, the application can also be by means of incorporating on conventional fluid absorption elements, such as for example a sanitary pad, in which case the carrier sheet having the reactive product does not need to include absorbent material.

In an embodiment the carrier sheet having the reactive product can include therein a protective cover made of a thin and permeable material such as a non-woven material which is characterized by its very fine structuring, such that it allows a close and perfect contact of the detector with the body surface of application, assuring a perfect functional effectiveness thereof, at the same time preventing any discomfort in use. In this case it is the protective cover which incorporates the adhesive impregnation for fixing the detector in the application use.

Said cover can be formed by means of two equal or different sheets, arranged above and below the carrier sheet having the reactive product, it can also be made by a single sheet folded and attached at the edge by means of an adhesive bead, for example by means of a hot-melt type polymer glue, whereby it is formed like a bag the inside of which the carrier sheet having the reactive product is inserted, the ends being closed by means of heat-welding.

A detector capable of providing an immediate response in the presence of moisture is thus obtained, giving rise to a warning effect for the person wearing it, with a signal which is shown by a thermal change, which is only detected by the user alone.

This detector therefore acts with complete discretion, allowing the user an effective ability for controlling the incontinence problem, without any type of repercussion with respect to other people, such that as a result of the privacy achieved it is also beneficial for the normal life and self-esteem of the users.

In this sense, with relation to female menstruation, the detector is useful for premenstrual use since it warns the user about the first leakage of the menstrual flow, allowing necessary measures to be taken so that stains visible through the clothing are not produced. But it is also useful for use during menstruation for women using a tampon as a retention means of the menstrual flow since in this case it warns the user when the tampon is saturated and a leakage occurs, so that, similarly, necessary measures are taken with the purpose of preventing stains visible through the clothing from being produced.

The use of this detector is also extremely comfortable since due to its flexibility and thinness it does not cause any impediment to the freedom of movements of the user, being able to be used equally both during the night and during daily activity; and it is applicable both with underwear (bras or slip), and with male or female swimsuits to provide security to person using them in any circumstance.

Said detector object of the invention therefore has very advantageous features, acquiring own identity and preferred feature for its intended function.

### Description of the Drawings

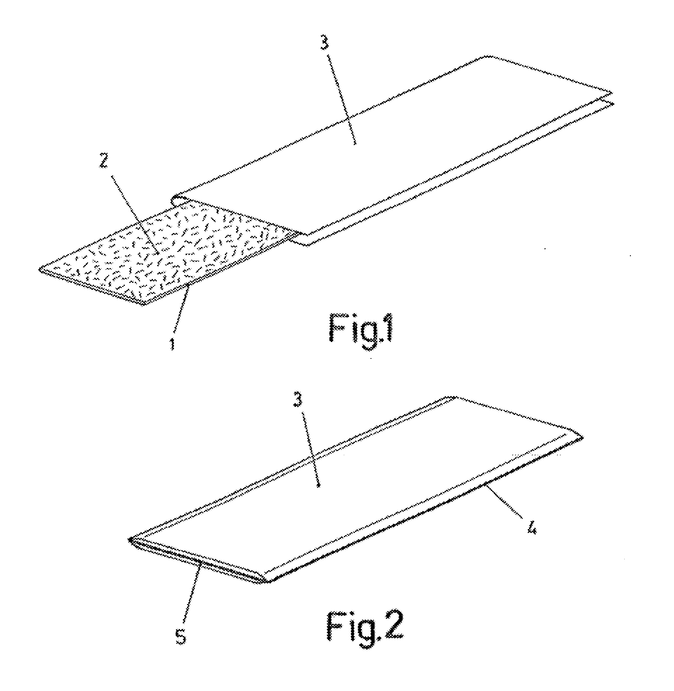

Figure 1 shows a practical embodiment of the detector object of the invention in an assembly arrangement.
Figure 2 is a perspective view of the closed detector arranged for use.

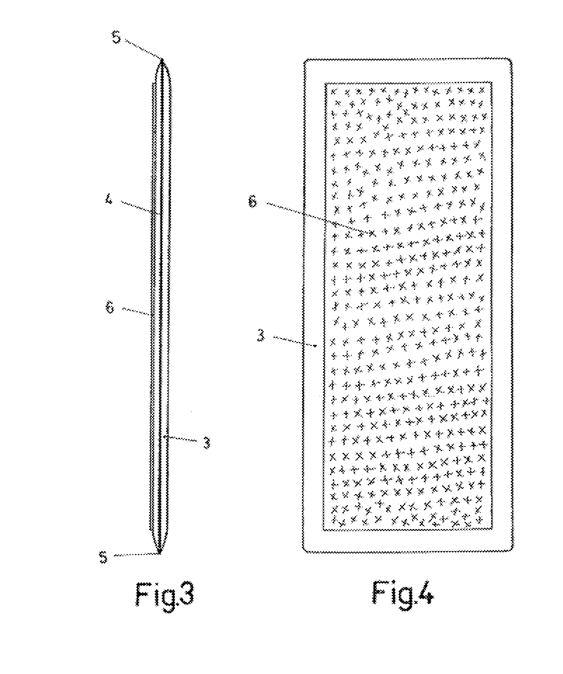

Figure 3 is a profile view of the previous detector.
Figure 4 is a corresponding view of the lower face of the detector.

### Detailed Description of the Invention

The object of the invention relates to a detector intended as prevention means for preventing the incontinence of bodily fluid such as urine, menstruation, etc., so that the people affected can realize at the time of the incontinence, in order to enable them to take the necessary measures immediately.

This detector consists of an assembly formed by a sheet element (1) provided with an amount of a product (2) thermally reactive with moisture such as sodium thiosulfate or any other product with the same reaction, which upon contacting with moisture reacts by absorbing heat, such that it gives rise to a cooling effect in the means where it is located.

The sheet element (1) is formed by a sheet made of impermeable and breathable material such as polyethylene, on which is spread on one or both faces the reactive product (2) by means of an arrangement which allows maintaining it distributed on the surface of said sheet (1).

To this end, the product (2) can be incorporated, for example, by means of housing in a distribution of compartmentalisations structured on the sheet (1) itself, by way of bags or meshes, a preferred embodiment being provided, by means of sprinkling the reactive product (2) on the sheet (1), such that the particles of the product (2) are retained between the fibres of the mentioned sheet (1), thus maintaining the distribution on the latter.

The sheet element (1) can further include an absorbent material, allowing the use of the detector in its application directly on the clothing; but the application can also be on conventional absorbent elements, such as for example, a sanitary pad in which case the sheet element (1) does not need to have an absorbent material.

In addition, the sheet element (1) provided with the reactive product (2) can incorporate adhesive impregnated areas for fixing it in the application use, an embodiment also being provided according to which on said sheet element (1) provided with the reactive product (2) there is arranged a covering (3) which can be formed by two equal or different sheets being incorporated above and below, with closing attachment between them on the outline, determining a closed assembly like a bag, the inside of which is housed the sheet element (1) provided with the reactive product (2).

According to a practical embodiment, the mentioned covering (3) is formed by means of a single sheet folded over itself and attached by the edges with an adhesive bead (4), for example by means of a hot-melt type polymer glue, the end being closed by means of an attachment (5) by heat-welding, without such closing solutions being limited.

The covering (3) is formed by a so-called non-woven material of the themobonded or spunbonded type, such that it determines an extremely fine and permeable microfibre structure, whereby the application of the detector on a body area results practically in a close contact of the reactive product (2) on the application surface, which gives rise to a complete reaction effectiveness of the product (2) since moisture is produced by any liquid flow in the application area.

In the lower outer part of the assembly thus formed, there is incorporated on the covering (3) an adhesive layer (6) on which a protective sheet of wax paper intended to be removed in the use application of the detector is provisionally incorporated such that the latter can be fixed by itself by means of the mentioned adhesive layer (6) on the clothing, both on underwear and on swimsuits, for the practical function of the application.

In the application use the detector is susceptible to be incorporated with respect to any body area which can be moistened with an incontinent body flow such that when the moisture is produced the product (2) of the detector reacts by absorbing heat whereby it consequently produces a cooling effect which can be detected by the user who will thus be aware of the flow leak with complete discretion, being able to adopt the necessary measures.

The structural assembly of the detector can adopt any configuration and variable dimensions, being very flexible and having a minimum thickness, it therefore does not at all affect the freedom of movements of the user and is perfectly adapted for incorporation on any garment, being able to be used comfortably both at night and during daily activity, including at pools or beaches.

## Claims

1. An incontinence detector of the type formed with a chemical which reacts with moisture producing a thermal effect capable of being detected by the user, **characterized in that** it comprises a sheet (1) of impermeable and breathable material on which is spread on one or both faces a reactive product (2), said sheet (1) being arranged inside an outer wrap covering (3) formed by a structured non-woven microfibre sheet material.

2. The incontinence detector according to claim 1, **characterized in that** the reactive product (2) is arranged in the carrier sheet (1) housed in a reticular distribution formed on said sheet (1).

3. The incontinence detector according to claim 1, **characterized in that** the reactive product (2) is arranged in the carrier sheet (1) by means of an impregnation by sprinkling, the particles of said product (2) being retained between the fibres of the sheet (1).

4. The incontinence detector according to claim 1, **characterized in that** the carrier sheet (1) having the reactive product (2) incorporates an absorbent material, allowing the application for use directly on clothing.

5. The incontinence detector according to claim 1, **characterized in that** according to one embodiment the wrap covering (3) is formed with a sheet folded over itself and closed like a bag by means of an adhesive bead between the long edges and heat-welding at the ends.
